(19) Europäisches Patentamt
European Patent Office
Office européen des brevets

(11) Veröffentlichungsnummer: 0 190 706
A2

(12) EUROPÄISCHE PATENTANMELDUNG

(21) Anmeldenummer: 86101377.9

(22) Anmeldetag: 03.02.86

(51) Int. Cl.⁴: C 07 D 495/04
H 01 M 4/60, H 01 B 1/12
//(C07D495/04, 339:00, 339:00)

(30) Priorität: 07.02.85 DE 3504144

(43) Veröffentlichungstag der Anmeldung:
13.08.86 Patentblatt 86/33

(84) Benannte Vertragsstaaten:
BE DE FR GB NL

(71) Anmelder: BASF Aktiengesellschaft
Carl-Bosch-Strasse 38
D-6700 Ludwigshafen(DE)

(72) Erfinder: Keller, Heimo, Prof.Dr.
Angelweg 28
D-6900 Heidelberg(DE)

(72) Erfinder: Heid, Renate, Dr.
Buchenweg 2
D-6803 Edingen-Neckarhausen(DE)

(54) Bis(ethylendithiolo)-tetrathiofuvalen-Derivat und seine Herstellung.

(57) 4, 5-Dimethyl-4', 5'-ethylendithiolo-tetrathiofulvalen sowie ein Verfahren zu dessen Herstellung, bei dem man 4, 5-Dimethyl-1,3-dithiolium-tetrafluoroborat und 4,5-Ethylendithiolo-1, 3-dithiolium-tetrafluoroborat mit Trialkylaminen umsetzt.

EP 0 190 706 A2

Croydon Printing Company Ltd.

Bis(ethylendithiolo)-tetrathiofulvalen-Derivat und seine Herstellung

Die Erfindung betrifft ein neues Bis(ethylendithiolo)-tetrathiofulvalen-Derivat sowie ein Verfahren zu seiner Herstellung.

Bis(ethylendithiolo)-tetrathiofulvalen ist bekannt z.B. aus S.S.P. Parkin et al. in Phys. Rev. Lett. 50, Seite 270 (1983). Die Radikalkationensalze derartiger Verbindungen haben unterhalb einer bestimmten Sprungtemperatur Supraleitfähigkeit.

Aufgabenstellung der Erfindung ist es, ein weiteres Derivat von Verbindungen dieser Klasse zu schaffen.

Die Aufgabe der Erfindung wird gelöst durch 4,5-Dimethyl-4',5'-ethylendithiolo-tetrathiofulvalen. Gegenstand der Erfindung ist außerdem ein Verfahren zur Herstellung dieser Verbindung. Weitere Gegenstände der Erfindung sind die Verwendung dieser Verbindungen als Leiter in der Elektrotechnik oder als Elektroden in elektrochemischen Primär- oder Sekundärelementen.

Das 4,5-Dimethyl-4',5'-ethylendithiolo-tetrathiofulvalen hat die Formel

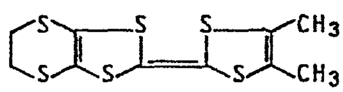

(I)

Diese Verbindung wird auch im folgenden mit der Kurzbezeichnung DIMET bezeichnet.

Zur Herstellung von DIMET setzt man 4,5-Dimethyl-1,3-dithiolium-tetrafluoroborat und 4,5-Ethylendithiolo-1,3-dithiolium-tetrafluoroborat mit Trialkylaminen um.

Das 4,5-Dimethyl-1,3-dithiolium-tetrafluoroborat hat die Formel

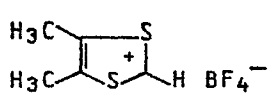

(II)

Das 4,5-Ethylendithiolo-1,3-dithiolium-tetrafluoroborat hat die Formel

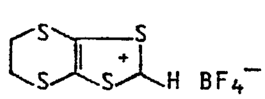

(III)

vG/P

Die Umsetzung von (II) und (III) zu DIMET (I) erfolgt mit tertiären Aminen vorteilhaft mit Diisopropylethylamin oder mit Triethylamin. Die Umsetzung wird zweckmäßig in Lösung vorgenommen, vorzugsweise im trocknen Acetonitril. Zweckmäßig arbeitet man unter Stickstoffatmosphäre im Bereich von -5°C bis +10°C, vorteilhaft um 0°C. Die Umsetzung kann jedoch auch in anderen Lösungsmitteln vorgenommen werden. Zur Isolierung des Produktes und der dabei erhaltenen Nebenprodukte fügt man zweckmäßig Wasser hinzu und saugt das ausgefallene rosafarbene Produktgemisch ab. Das DIMET (I) läßt sich aus dem Reaktionsgemisch durch chromatographische Trennung isolieren.

Zur Herstellung der Verbindung der Formel (II) geht man von dem 4,5-Dimethyl-1,3-dithiol-2-thion aus, das man zunächst methyliert. Die Umsetzung erfolgt zweckmäßig mit Methyljodid in Nitromethan. Man erhält das 4,5-Dimethyl-2-S-methyl-1,3-dithioliumjodid. Diese Verbindung wird anschließend reduziert. Die Reduktion erfolgt zweckmäßig mit Natriumborhydrid in Methanol, so daß man die Verbindung 4,5-Dimethyl-2-S-methyl-1,3-dithiol erhält. Diese Verbindung wird anschließend mit Tetrafluoroborsäure in Acetanhydrid zum 4,5-Dimethyl-1,3-dithiolium-tetrafluorborat (II) umgesetzt.

Zur Herstellung des zweiten Reaktionspartners (III) geht man vom 4,5-Ethylendithiolo-1,3-dithiol-2-thion aus, das man zunächst mit Dimethoxycarboniumtetrafluoroborat alkyliert. Man erhält somit das 4,5-Ethylendithiolo-2-S-methyl-1,3-dithioliumtetrafluoroborat. Diese Verbindung wird anschließend in Acetonitril mit Natriumborhydrid reduziert zu 4,5-Ethylendithiolo-2-S-methyl-1,3-dithiol. Aus dieser Verbindung erhält man durch Umsetzung mit Tetrafluoroborsäure in Acetanhydrid die Verbindung der Formel (III).

Die Verbindung DIMET (I) kann wie oben angegeben durch Umsetzung mit Trialkylaminen erhalten werden.

DIMET (I) läßt sich mit gutem Erfolg als Elektronendonor verwenden. Hierzu wird DIMET elektrochemisch in einer Elektrolytlösung, die Leitsalze enthält, oxidiert. Dazu bringt man DIMET in eine U-förmige Zelle, die mit Hilfe einer Fritte in einen Anoden- und Kathodenraum getrennt ist, und nimmt Platinbleche als Elektroden. Als Elektrolyt verwendet man zweckmäßig Methylenchlorid oder Tetrahydrofuran. Es können die üblichen Leitsalze verwendet werden. Die erhaltenen DIMET-Kationensalze haben vorzügliche elektrische Eigenschaften. Sie können beispielsweise als Material für elektrische Leiter oder als Material für Elektroden dienen. Besonders zu beachten ist die Supraleitfähigkeit. Man kann die Materialien auf Trägermaterial aufbringen, das zweckmäßig aus elektrisch leitfähigen Werkstoffen besteht. Dies sind z.B. Metalle in Faser- oder Gitterform oder Graphit in Faser- oder Gitterform oder die bekannten elektrisch leitfähigen Polymerisate. Es können aber auch die Donorkationensalze mit anderen leitfähigen Verbindungen verpreßt werden, so z.B. mit Graphit. Es ist ein besonderer Vorzug, daß die so erhaltenen Materialien in Primär- oder Sekundärbatterien als Elektrode Verwendung finden können.

Beispiel 1

Herstellung von (II)

a) Herstellung von 4,5-Dimethyl-2-S-methyl-1,3-dithioliumjodid

2,65 g 4,5-Dimethyl-1,3-dithiol-2-thion werden in 122 ml Nitromethan (mit $CaCl_2$ getrocknet) gelöst, mit 5,6 ml Methyljodid versetzt und 24 Stunden stehen lassen. Es findet ein Farbumschlag von gelb nach orangerot statt. Das alkylierte Thion wird mit viel trockenem $CCl_4$ gefällt, abgesaugt und ohne weitere Reinigung umgesetzt. Das NMR-Spektrum in $CDCl_3$ zeigt zwei Singuletts bei $\delta$ = 2,7 ppm (6H) und bei $\delta$ = 3,3 ppm (3H).

b) Herstellung von 4,5-Dimethyl-2-S-methyl-1,3-dithiol

7,2 g (0,024 mol) des alkylierten Thions werden in 250 ml abs. Methanol gelöst, auf $0^{o}C$ abgekühlt und durch langsame, portionsweise Zugabe von 4,5 g Natriumborhydrid reduziert. Die Reaktion läuft unter Aufschäumen und Erwärmen ab, wobei sich die Lösung von gelb nach nahezu farblos entfärbt. Nach zweistündigem Nachrühren gibt man 500 ml abs. Ether zur Fällung des gebildeten Natriumjodids hinzu und läßt über Nacht bei $-18^{o}C$ stehen. Dann wird in einen 2l-Schütteltrichter filtriert und bis zur Phasentrennung mit Wasser versetzt. Man wäscht die organische Phase mit Wasser, trocknet über $MgSO_4$, filtriert und destilliert das Lösungsmittel ab.

Zurück bleiben 3,98 g (94 % Ausbeute) eines gelb-orangefarbenen Öls, das ohne weitere Reinigung möglichst schnell in der nächsten Reaktionsstufe eingesetzt wird.

c) Herstellung des 4,5-Dimethyl-1,3-dithiolium-tetrafluoroborat (II)

Zu einer gekühlten (Eis/Salz-Mischung) Lösung von 3,98 g (0,22 mol) der nach b) erhaltenen Verbindung in 13,2 ml Acetanhydrid tropft man langsam unter Rühren 4,4 g 48 %ige Tetrafluoroborsäure unter Inertgas. Mit etwas Verzögerung läuft dann die Reaktion sehr heftig ab! Man läßt wenige Minuten nachrühren, fällt dann mit ca. 100 ml abs.

Ether das Produkt aus, saugt ab und wäscht das nahezu weiße Dithioliumsalz mit abs. Ether. Die Ausbeute beträgt 3,38 g (70 %).

Das NMR-Spektrum in $CD_3CN$ zeigt zwei Singuletts bei $\delta$ = 2,8 ppm (6H) und bei $\delta$ = 10,85 ppm (1H). Die erhaltene Verbindung (II) wird wie unter g) beschrieben weiter umgesetzt.

d) Herstellung von III

Dimethoxycarboniumtetrafluoroborat

Für die Alkylierung von 4,5-Ethylendithiolo-1,3-dithiol-2-thion wurde zunächst Dimethoxycarboniumtetrafluoroborat präpariert und ohne Isolierung weiterverarbeitet.

In einer Ar-Atmosphäre wird bei -30°C eine Lösung von 7,3 ml $BF_3$-Etherat (56 mmol) in 6 ml trockenem $CH_2Cl_2$ innerhalb von 5 Minuten zu 5,5 m l (50 mmol) Trimethylorthoformiat in einem 2l Dreihalskolben getropft. Man bringt die Lösung auf 0°C, rührt 15 Minuten, kühlt auf -70°C, saugt die Mutterlauge von dem weißen Produkt ab, digeriert mit 5 ml Methylenchlorid und saugt nochmals ab. Die Ausbeute an weißem kristallinem Dimethoxycarboniumsalz wird auf 85 % geschätzt.

Alkylierung

Das gebildete Alkylierungsmittel wird in 240 ml trockenem Methylenchlorid suspendiert. Dazu tropft man bei -20°C 8 g 4,5-Ethylendithiolo-1,3-dithiol-2-thion in 500 ml trockenem $CH_2Cl_2$, wobei sich die Reaktionslösung allmählich von gelb nach orangebraun verfärbt. Unter Rühren läßt man Auftauen und über Nacht stehen. Dann filtriert man die jetzt rote Lösung und fällt mit ca. 1l $CCl_4$ unter Eiskühlung das Reaktionsprodukt aus. Nach dem Absaugen und Trocknen erhält man 9,46 g (82 %) eines orangefarbenen Produktes. Umfällen aus Methylenchlorid mit $CCl_4$ ergibt lange orangefarbene glänzende Nadeln vom Schmp. 125-126°C.
Elementaranalyse: Summenformel: $C_6H_7BF_4S_5$ Mr = 326,25

| | C | H | S |
|---|---|---|---|
| Ber. | 22,09 | 2,16 | 49,14 |
| Gef. | 21,94 | 2,19 | 49,23 |

e) Herstellung von 4,5-Ethylendithiolo-2-S-methyl-1,3-dithiol

So wurden 8 g der nach d) hergestellten Verbindung in 160 ml trockenem $CH_3CN$ bei Raumtemperatur gelöst und mit 2,4 g $NaBH_4$ in kleinen Portionen versetzt. Die Lösung entfärbt sich allmählich von dunkelrot nach hellgelb und erwärmt sich auf ca. 40°C. Das Ende der Reaktion erkennt man daran, daß bei weiterer $NaBH_4$-Zugabe die Temperatur nicht mehr ansteigt, sondern allmählich zurückgeht. Man verdünnt die Reaktionsmischung mit Wasser, engt sie ein, extrahiert den Rückstand mehrmals mit Ether, trocknet über $MgSO_4$ und destilliert den Ether ab. Zurück bleibt ein gelbes Öl in über 90 % Ausbeute.

f) Herstellung von 4,5-Ethylendithiolo-1,3-dithiolium-tetrafluoroborat (III)

4,18 g der nach e) erhaltenen Verbindung werden in 10,5 ml Acetanhydrid und 2,5 ml abs. Ether gelöst und unter Rühren in Stickstoffatmosphäre auf 0°C gekühlt. Bei dieser Temperatur tropft man 3,5 g 48 %ige Tetrafluoroborsäure hinzu, läßt wenige Minuten nachrühren und gibt dann 80 ml abs. Ether zu der Reaktionsmischung. Das ausgefallene gelbbraune Dithioliumsalz wird abgesaugt und mit abs. Ether gewaschen.

Ausbeute: 2,69 g ≙ 55 %.

g) Herstellung von DIMET (I)

540 mg (1,9 mmol) von (III) und 420 mg (1,9 mmol) von (II) werden in 10 ml trockenem Acetonitril gelöst und unter Rühren und Stickstoffatmosphäre auf 0°C gekühlt. Bei dieser Temperatur tropft man zügig 4,6 ml Diisopropylethylamin hinzu, läßt zehn Minuten nachrühren, gibt ca. 200 ml Wasser dazu, saugt das ausgefallene rosafarbene Produkt ab und trocknet es im HV. Man erhält 550 mg Rohausbeute (90 %).

Man kocht die 550 g Rohprodukt mit zweimal 45 ml und einmal 30 ml Acetonitril auf und filtriert ab. Zurück bleibt ein fleischfarbenes Pulver. Das Filtrat, das DIMET (I) sowie stark gefärbte Verunreinigungen enthält, wird einrotiert, der Rückstand (= 450 mg) in je 20 ml trockenem $CH_2Cl_2$ und n-Hexan gelöst und über eine Kieselgelsäule (90 cm Länge; 4 cm Durchmesser) mit einem Gemisch aus Methylenchlorid und n-Hexan im Verhältnis 1:1 als Laufmittel chromatographiert. Die optimale Fließgeschwindigkeit der Säule liegt bei 6-7 ml/min. Nach der ersten Verbindung und einer kleinen Übergangsfraktion, kommt DIMET (I) von der Säule.

Das so erhaltene DIMET (I) wird durch Umkristallisation aus Acetonitril weiter gereinigt und anhand der Elementaranalyse, des NMR-Spektrums und des Massenspektrums charakterisiert.

Elementaranalyse: Summenformel $C_{10}H_{10}S_6$ $M_r$ = 322,57

| | C % | H % | S % |
|---|---|---|---|
| Ber. | 37,24 | 3,12 | 59,64 |
| Gef. | 37,5 | 2,88 | 59,86 |

Das NMR-Spektrum der Verbindung in $CDCl_3$ zeigt die beiden für diese Substanz erwarteten Singuletts bei $\delta$ = 1,9 ppm (6H) und bei $\delta$ = 3,25 ppm (4H), neben kleinen Peaks, die von Verunreinigungen aus dem $CDCl_3$ herrühren.

Beispiel 2

Zyklisches Voltagramm von DIMET (I)

Das zyklische Voltagramm in dem System $CH_2Cl_2/Bu_4N^+BF^-_4$ zeigt zwei Einelektronen-Redoxschritte bei 0,44 Volt und bei 0,83 Volt. Außerdem findet man eine irreversible Oxidation oberhalb 2,04 Volt.

Beispiel 3

Elektrokristallisation mit DIMET (I) als Donor

DIMET (I) wird in einer kleinen U-Rohr-Zelle, in der Anoden- und Kathodenraum durch eine Fritte getrennt sind, mit Platinblechelektroden, mit und ohne Inertgas elektrochemisch umgesetzt. Die Elektrolyse-Lösungen in Methylenchlorid und vor allem in THF sind 0,5 x $10^{-3}$ bis 5,0 x $10^{-3}$ molar an Donor, während die Leitsalzkonzentrationen von 0,01 bis 0,3 molar variiert wurden, wobei $Bu_4N^+X^-$ mit $X^-$ = $PF^-_6$, $ReO^-_4$, $I^-_3$, $AsF^-_6$, $ClO^-_4$ und $NO^-_3$ als Leitsalz eingesetzt werden. Man elektrolysiert sowohl mit konstanter Spannung, die zwischen 0,45 und 2,0 Volt variiert wird, was Stromflüsse 0,45-2,0 V zwischen 5 bis 40 $\mu$A bedingt, als auch mit konstanter Stromstärke zwischen 5 und 40 $\mu$A, woraus in der Regel Spannungen von 0,45 bis 2 Volt resultieren.

In allen Fällen erhält man schwarze Kristalle in Form von Nadeln oder Platten. Sie fallen bei Elektrolysen in Methylenchlorid erst nach langsamem Abdunsten der Anodenraumflüssigkeit an, während in THF als Lösungsmittel die Kristalle von der Anode wegwachsen. Sie werden abgesaugt, mit THF gewaschen und zunächst unter dem Mikroskop betrachtet. Man sieht folgende Kristallformen:

| | |
|---|---|
| $(DIMET)_x(PF_6)_y$ : | (aus $CH_2Cl_2$) schwarze Nädelchen und Blättchen |
| $(DIMET)_x(PF_6)_y$ : | (aus THF) große schwarze Nadeln ca. 0,8 cm |
| $(DIMET)_x(I_3)_y$ : | kleine schwarze stark glänzende Rauten aus THF |
| $(DIMET)_x(ReO_4)_y$: | ca. 0,5 cm lange schwarze Nadeln |
| $(DIMET)_x(AsF_6)_y$: | schwarze glänzende Nadeln mit seitlichen Auswüchsen |
| $(DIMET)_x(NO_3)_y$ : | schwarze glänzende rechteckige Flächen |
| $(DIMET)_x(ClO_4)_y$: | schwarze glänzende lange rechteckige Flächen. |

Patentansprüche

1. 4,5-Dimethyl-4',5'-ethylendithiolotetrathiofulvalen.

2. Verfahren zur Herstellung von 4,5-Dimethyl-4',5'-ethylendithiolotetrathiofulvalen, dadurch gekennzeichnet, daß man 4,5-Dimethyl-1,3-dithiolium-tetrafluoroborat und 4,5-Ethylendithiolo-1,3-dithiolium-tetrafluoroborat mit Trialkylaminen umsetzt.

3. Verwendung von Verbindungen nach Anspruch 1 als Material für elektrische Leiter oder Elektroden.